# EUROPEAN PATENT APPLICATION

(11) **EP 1 684 488 A2**
(43) Date of publication of application: **26.07.2006**
(21) Application number: 06100367.9
(22) Date of filing: 16.01.2006
(51) Int. Cl.: H04L 29/06, A61B 5/00

(54) **System, performance monitor, server, and computer program**

(30) Priority: 19.01.2005 FI 20055027
(71) Applicant: Polar Electro Oy, 90440 Kempele (FI)
(72) Inventor: Sorvisto, Mika, 90440 Kempele (FI)
(74) Representative: Antila, Harri Jukka Tapani

(57) **Abstract**

The invention relates to a system, performance monitor, server and computer product. The system comprises at least one user-specific performance monitor for registering training information, where the performance monitor communicates training information using a two-way wireless data transmission connection and is associated with a performance monitor-specific identifier. The system further comprises at least one server for managing the performance monitor, where the server communicates training information with the user-specific performance monitor using a two-way wireless data transmission connection and performance monitor-specific identifiers. The server is in a first stand-by mode for a pre-determined time window of the server for establishing the two-way wireless data transmission connection, and the performance monitor is in a second stand-by mode for a pre-determined time window of the performance monitor for establishing the two-way wireless data transmission connection, where the predetermined time window temporally overlaps with the predetermined time window of the server.

## Description

### FIELD

The invention relates to a system for processing training information on a user group of performance monitors, a user-specific performance monitor, a server for managing a group of performance monitors, and a computer program for managing a group of performance monitors.

### BACKGROUND

A user-specific performance monitor can be employed for registering a physical performance of a performance monitor user and/or ambient conditions during use. If desired, the information registered in the performance monitor can be transmitted to a device external to the performance monitor, where information related to the use of the performance monitor can be stored and processed.

In some group applications, such as team sports and physical education at schools, a group is given user-specific performance monitors which, when used, register user-specific information on each user. User-specific information can be transmitted to an external device, which is typically managed by the person in charge for the group. The management of a group of performance monitors and the transmission of user-specific information on a large group of users between the group of performance monitors and the external device is difficult from the point of view of the person in charge. For this reason, it is necessary to examine technology for external management of a performance monitor.

### BRIEF DESCRIPTION

The object of the invention is to provide a system, a user-specific performance monitor, a server and a computer program so as to enable easy external management of a performance monitor.

A first aspect of the invention relates to a system for processing training information on a user group of performance monitors, comprising: at least one user-specific performance monitor for registering training information, the at least one performance monitor comprising a performance monitor communication unit, which is configured to communicate training information using a two-way wireless data transmission connection, and at least one performance monitor-specific identifier being associated with the at least one user-specific performance monitor; and at least one server for managing the performance monitor, the at least one server comprising a server communication unit, which is configured to communicate training information with at least one user-specific performance monitor using a two-way wireless data transmission connection and performance monitor-specific identifiers, the server communication unit being configured to be in a first stand-by mode for a predetermined time window of the server for establishing the two-way wireless data transmission connection; and the performance monitor communication unit being configured to be in a second stand-by mode for a pre-determined time window of the performance monitor for establishing the two-way wireless data transmission connection, the predetermined time window of the performance monitor temporally overlapping at least partly with the predetermined time window of the server.

A second aspect of the invention relates to a user-specific performance monitor for registering training information on a user group, the user-specific performance monitor belonging to a group of performance monitors and comprising a performance monitor communication unit, which is configured to communicate training information with a server managing the group of performance monitors using a two-way wireless data transmission connection, and a performance monitor-specific identifier being associated with the user-specific performance monitor to distinguish the user-specific performance monitor from the other performance monitors, the performance monitor communication unit being configured to be in a second stand-by mode for a predetermined time window of the performance monitor for establishing the two-way wireless data transmission connection, the predetermined time window of the performance monitor temporally overlapping at least partly with a predetermined time window of the server, the predetermined time window of the server being the time the server is in a first stand-by mode for establishing the two-way data transmission connection.

A third aspect of the invention relates to a server for managing a group of performance monitors, comprising a server communication unit configured to communicate training information with a user-specific performance monitor of the group of performance monitors using a two-way wireless data transmission connection and a performance monitor-specific identifier, the server communication unit being configured to be in a first stand-by mode for a predetermined time window of the server for establishing the two-way data transmission connection, the predetermined time window of the server temporally overlapping at least partly with a predetermined time window of the performance monitor, the predetermined time window of the performance monitor being the time the user-specific performance monitor is in a second stand-by mode for establishing the two-way wireless data transmission connection.

A fourth aspect of the invention relates to a computer software product for managing a group of performance monitors, the computer software product being incorporated into a distribution medium and including encoded instructions for communicating training information between a user-specific performance monitor of the group of performance monitors and a server using a two-way wireless data transmission connection and performance monitor-specific identifiers, the computer software product further comprising instructions for keeping the performance monitor in a second stand-by mode for a predetermined time window of the performance monitor, the predetermined time window of the performance monitor temporally overlapping at least partly with a predetermined time window of the server, the predetermined time window of the server being the time the server is in a first stand-by mode for establishing the two-way wireless data transmission connection.

A fifth aspect of the invention relates to a computer software product for managing a group of performance monitors, the computer software product being incorporated into a distribution medium and including encoded instructions for communicating training information between a user-specific performance monitor of the group of performance monitors and a server using a two-way wireless data transmission connection and performance monitor-specific identifiers, the computer software product further comprising instructions for keeping the performance monitor in a first stand-by mode for a predetermined time window of the server, the predetermined time window of the server temporally overlapping at least partly with a predetermined time window of the performance monitor, the predetermined time window of the performance monitor being the time the user-specific performance monitor is in a second stand-by mode for establishing the two-way wireless data transmission connection.

Preferred embodiments of the invention are disclosed in the dependent claims.

The invention is based on defining predetermined moments for a server and a performance monitor of a group of performance monitors when the server and the performance monitor are in a stand-by mode for establishing a data transmission connection.

The method and system according to the invention provide several advantages. One advantage is power savings in the performance monitor and server because the stand-by mode can be timed to the moment when there is a need for a data transmission connection. Furthermore, the invention enables transmission of timed training information.

### LIST OF FIGURES

The invention will now be described in greater detail by means of preferred embodiments with reference to the accompanying drawings, in which
Figure 1 illustrates an example of the structure of a system,
Figure 2A illustrates a first example of the structure of a performance monitor,
Figure 2B illustrates a second example of the structure of a performance monitor,
Figure 3 illustrates an example of the structure of a server,
Figure 4 illustrates a first example of timing time windows,
Figure 5 illustrates a second example of timing time windows,
Figure 6 illustrates an example of timing data transmission connections.

### DESCRIPTION OF EMBODIMENTS

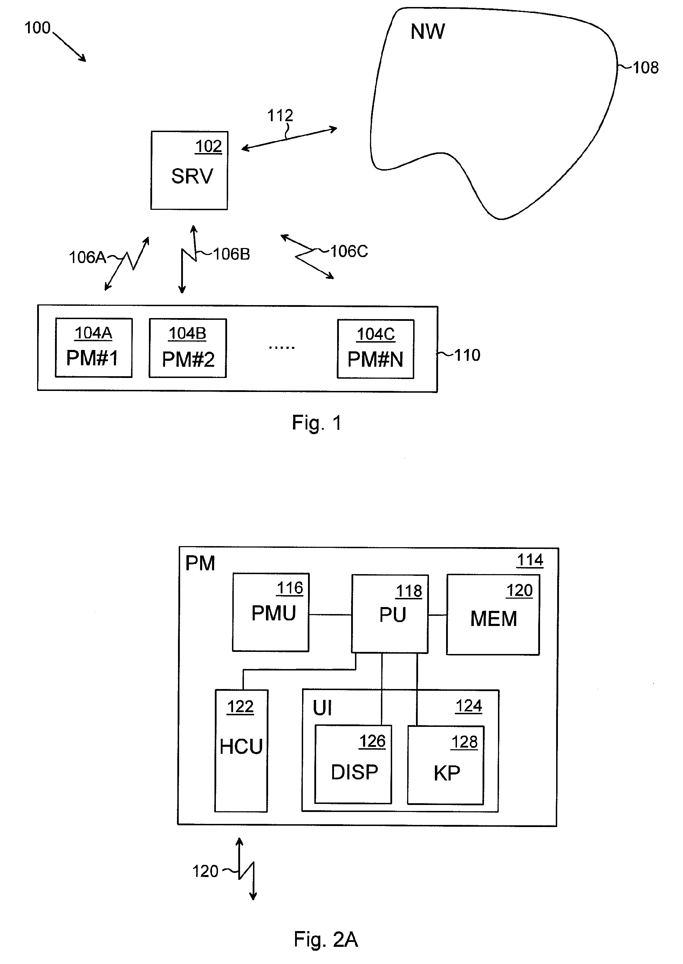

Figure 1 illustrates a system 100 for processing training information on a user group of performance monitors, the system comprising a server (SRV) 102 and a group of performance monitors consisting of N user-specific performance monitors (PM#1, PM#2, PM#N) 104A, 104B, 104C where N ≥ 1. The user-specific performance monitor 104A to 104C may be in a two-way wireless data transmission connection 106A, 106B, 106C with the server 102.

The two-way wireless data transmission connection 106A to 106C is a connection where the server 102 may transmit signals to a user-specific performance monitor 104A to 104C and receive signals from the user-specific performance monitor 104A to 104C over a wireless interface and where the user-specific performance monitor 104A to 104C may transmit signals to the server 102 and receive signals from the server 102 over a wireless interface. For the sake of simplicity, the two-way wireless data transmission connection 106A to 106C is called a data transmission connection and referred to by the same reference numbers.

The data transmission connection 106A to 106C is typically a local area connection having a maximum range of 5 to 10 meters, for example, without being limited thereto.

The user-specific performance monitor 104A to 104C is typically an electronic device which is carried by the user and which registers the user's training information.

The training information is typically information which characterizes a performance of the performance monitor user and/or by which the user's performance can be controlled. The user's performance may be a physical performance and/or a mental performance.

Training information may be generated by measuring the user's electrocardiogram, for example, and by forming variables characterizing the heart rate from the measured electrocardiogram, such as the heart rate interval between successive heart beats.

Using the heart rate interval, the user-specific performance monitor 104A to 104C may generate variables characterizing the user's physiology, such as heart rate frequency, resting heart rate, stress parameter, energy consumption and other secondary variables.

In some embodiments, training information is generated by measuring the user's physical activity by means of acceleration sensors, for instance. In addition, the user-specific performance monitor 104A to 104C may measure and register training information related to the use environment, such as the pressure and/or temperature. In this context, the user-specific performance monitor 104A to 104C is briefly referred to as a performance monitor 104A to 104C.

The training information may comprise following elements, for instance:
- user information, such as the user's initial weight, height, gender and age
- the user's target weight
- the user's current weight
- the user's target for energy intake
- the user's target for energy consumption
- the user's energy intake
- the energy consumed by the user
- the form of physical activity
- an entry into a training diary
- the target value for a heart rate variable, such as the resting heart rate or the maximum heart rate
- a limit value for a heart rate variable
- a limit value for a variable representing performance
- another variable inputted by the user, such as waist measurement or number of exercises
- the user's location information
- the user's activity

The user group of the performance monitors 104A to 104C comprises users each of whom has a performance monitor 104A to 104C available and whose training information the performance monitors 104A to 104C register. The user group is typically a school class, an army contingent, a sports team or another group which carries out performances, such as exercises and/or sports at the same time. The solution described is not limited to the above-mentioned cases.

In an embodiment, the user group includes one user.

Each performance monitor 104A to 104C is associated with a performance monitor-specific identifier, on the basis of which the server can distinguish the training information to be received from each performance monitor 104A to 104C and/or the training information to be transmitted to each performance monitor 104A to 104C. The identifier may be a code, which is transmitted on the data transmission connection 106A to 106C and which may be included in the data stream that transfers training information. The identifier may also be included in the radio channel used by the data transmission connection 106A to 106C by, for example, allocating a performance monitor-specific radio frequency and/or time frame to each data transmission connection 106A to 106C. The identifier may also be a personal code which is fed by the user, is previously known to the server and on the basis of which both the user and the performance monitor 104A to 104C can be distinguished from the other users and performance monitors 104A to 104C. In an embodiment, the identifier is a code generated from the user information. Such user information may include gender, age, weight and/ or a combination thereof. The solution described is not, however, restricted to the above-mentioned identifier types.

The server 102 may be, for example, a personal computer, a hand-held computer (PDA, Personal Digital Assistant), a mobile phone, a performance monitor or any electronic device that supports the data transmission connection 106A to 106C and includes means for managing performance monitors 104A to 104C. In an embodiment, the server is an internal or an external module, which is connectable to a computer and includes a processor and memory enabling server 102 functionalities. In this context, the management refers to the monitoring and/or controlling of the performance monitors 104A to 104C.

The server 102 is typically operated by an operator, who may be the instructor or coach of a sports group, for example.

In an embodiment, the server 102 may be provided with a network connection 112 to an external network (NW) 108, such as a mobile communication system and/or the Internet. The mobile communication system is, for example, a GSM system (Global System for Mobile Communications), a UMTS system (Universal Mobile Telecommunications System) or a CDMA2000 system. In that case, the training information included in the server 102 can be transferred over the external network to a desired address through the email, for example. The solution described is not, however, restricted to the above-mentioned mobile communication systems.

The data transmission connection 106A to 106C is typically a radio connection but not restricted thereto.

In an embodiment, the data transmission connection 106A, 106B, 106C is a Bluetooth connection.

In an embodiment, the data transmission connection 106A, 106B, 106C is an infrared connection, such as IrDA (Infrared Data Association).

In some applications, the system 100 can be used so that at the beginning of a training session or a similar time period common to a user group, performance monitors 104A to 104C are handed out to the users. In that case, each user can be associated with the identifier of each performance monitor 104A to 104C, and thus the system can associate each person with the correct performance monitor 104A to 104C and/or training information. Association can be performed by logically combining the user's name or another user identifier with a physical identifier of the performance monitor 104A to 104C, such as the number of the performance monitor 104A to 104C. The users' names and the numbers of the performance monitors may be preprogrammed in the server 102, or they can be programmed in the server at the beginning of a training session.

In an embodiment, the performance monitors 104A to 104B are in the users' possession also outside the training session.

During use, each performance monitor 104A to 104C registers training information on its user and optionally performs other functions, such as informs the user of the current heart rate and/or activity in relation to the set limits.

In an application of the system, the users are expected to return the performance monitors 104A to 104C at the end of the training session or at another moment to the vicinity of the server 102. There may be storage means in connection with the server 102, such as a bag, or another structure where the performance monitors 104A to 104C can be stored and optionally transported.

Figure 2A illustrates an example of the structure of a performance monitor 114. The performance monitor 114 comprises a performance sensor 116, a processing unit 118, a memory unit 120 and a performance monitor communication unit 122. In an embodiment, the performance monitor 114 comprises a user interface 124, which may include a keypad 128 and/or a display 126, for instance. The performance monitor 114 may be a device which is arranged on the user's wrist or around the chest. As regards the solution described, the location of the performance monitor 114 on the user's body is not an essential feature per se.

The performance sensor 116 measures a measurement value needed to determine a variable characterizing the performance, such as the acceleration, user's temperature, and/or part of the electrocardiogram. The variable characterizing the performance can be determined in the processing unit 118 by calculations, for example, using computer software stored in a memory unit 120. The performance sensor 116 may be integrated into the performance monitor 114. In an embodiment, the performance sensor 116 is an external module that can be attached to the performance monitor 114 and optionally detached from it.

The processing unit 118 may also process training information.

In an embodiment, the performance monitor 114 includes an operating system with open interfaces.

The processing unit 118 may be implemented by analogue circuits, ASIC circuits (Application Specific Integrated Circuit), a digital processor, memory and computer software. The processing unit 118 may form part of the computer of the performance monitor 114.

At least part of the training information can be stored in the memory unit 120 connected to the processing unit 118. In addition, the memory unit 120 may include encoded instructions for executing a computer process in the processing unit 118. In addition to this, performance monitor-specific identifiers can be stored in the memory unit 120.

The user interface 124 typically includes a display unit 126 and a screen driver. The display unit 126 may include LCD components (LCD, Liquid Crystal Display), for instance.

The user interface 124 may further comprise a keypad 128, which the user may use to feed commands into the performance monitor 114.

The performance monitor communication unit 122 implements a data transmission connection 234, which corresponds to the data transmission connection 106A to 106C of Figure 1.

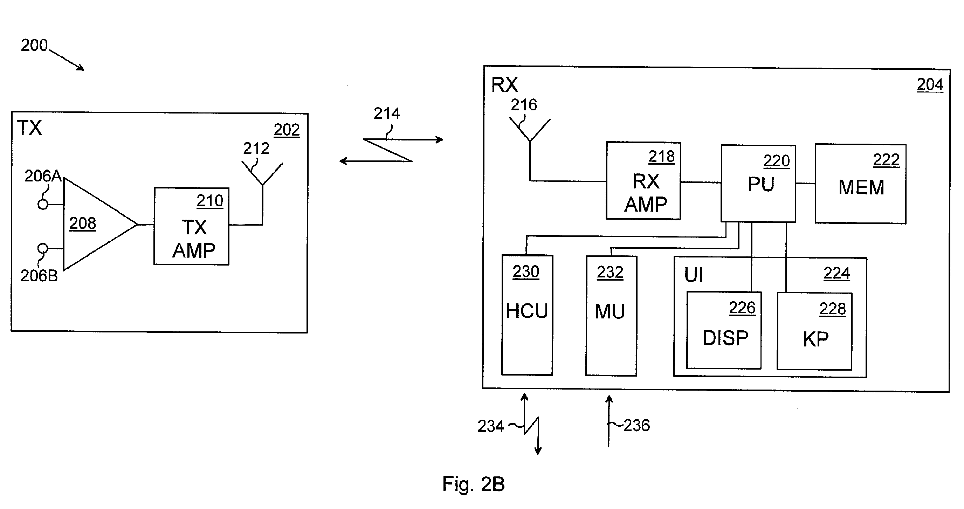

Figure 2B illustrates an embodiment of the solution disclosed where the performance monitor 200 is a heart rate meter. In this case, the performance monitor 200 comprises means for measuring the heart rate. The performance monitor 200 typically comprises electrodes 206A, 206B, an ECG preamplifier 208 (ECG, electrocardiogram) provided with differential input terminals, a transmitter amplifier (TX AMP) 210, a transmitter antenna 212, a receiver antenna 216, a receiver amplifier (RX AMP) 218, a processing unit (PU) 220, a memory unit (MEM) 222 and a user interface (UI) 224.

The parts 206A to 212 of the performance monitor illustrated in Figure 2B may in some embodiments of the performance monitor 200 be included in the transmitter unit 202, which is typically arranged around the user's chest. Parts 216 to 224 of the performance monitor 200 may be included in the processing unit 204, which may be a wrist device worn on the user's wrist or a device to be connected to the structure of a bicycle.

The electrodes 206A, 206B detect the electric potential generated by the electric activity of the heart muscle and produce an ECG signal characterizing the electric activity of the heart muscle. The ECG signal is fed from the electrodes 206A, 206B into the ECG preamplifier 208.

The ECG preamplifier 208 preamplifies the ECG signal and transmits the preamplified ECG signal to the transmitter amplifier 210. The transmitter amplifier 210 may include a plurality of successive amplifier stages, such as an AGC amplifier (AGC, Automatic Gain Control) and a power amplifier.

The amplified ECG signal is fed into the transmitter antenna 212, which generates an electromagnetic field 214 for transferring ECG data. The ECG data may comprise the ECG as such, for example, part of the ECG and/or timing information on the heart rate. The timing information may include a timing pulse, which represents the timing of a predetermined part of the ECG.

The timing information may be determined by identifying a QRS complex of the ECG and by determining the timing of the QRS complex. The QRS complex may be detected by a pulse detector, for example. The transmitter unit 202 may generate a burst which corresponds to the timing of each pulse, for example, and is transmitted to the processing unit 204. The processing unit 204 receives the bursts and can determine the heart rate interval between successive bursts.

The receiver antenna 216 detects the electromagnetic field 214 generated by the transmitter antenna 212, thus producing an induced electric signal, which is fed into the receiver amplifier 218.

The receiver amplifier 218 processes the electric signal by filtering and amplifying it, for example. The receiver amplifier 218 may comprise several successive regulation stages.

The receiver amplifier 218 feeds the electric signal into the processing unit 220, which typically performs analogue signal processing on the electric signal, such as filtering and analog-to-digital conversions. The processing unit 220 may further perform digital processing, such as digital filtering, signal formatting, ECG signal detection and ECG signal analysis.

The processing unit 220 may determine the value of the heart rate variable characterizing the heart rate. The heart rate variable may be a heart rate interval, a heart rate frequency, variation in the heart rate interval and/or variation in the heart rate frequency.

The transmitter unit 202 illustrated in Figure 2B typically includes components 206A to 212, measures the ECG and transmits the ECG information to the processing unit 204. In some embodiments, the transmitter unit 202 may comprise a heart rate detector, which detects a predetermined part of the ECG, generates a transmitter burst representing timing of a predetermined part of the ECG and/or a bit stream, and transmits the transmitter burst to the processing unit 204.

The processing unit 204 typically comprises components 216 to 128, which process the electric signal used in wireless data transmission and ECG information and produce a user interface.

In an embodiment, the transmitter unit 202 and the processing unit 204 are integrated into the same performance monitor, which can be used as a belt around the chest, for example. In that case, some of the components illustrated in Figure 2B, such as antennas 212, 216 and amplifiers 210, 218, may not be needed.

The example of Figure 2B further illustrates a performance monitor communication unit (HCU) 230 and measuring unit (MU) 232. The performance monitor communication unit 230 implements a data transmission connection 234, which corresponds to the data transmission connection 106A to 106C of Figure 1. The performance monitor communication unit 230 communicates training information with the server 102. The performance monitor communication unit 230 can be controlled by the processing unit 220, and the performance monitor communication unit 230 may use the memory capacity of the memory unit 222 for storing communication parameters that define the data transmission connection 234, for example.

The measuring unit 232 may measure an ambient variable 236, such as the temperature or pressure. The performance monitor 200 may comprise several measuring units 232 for measuring various ambient variables. The measuring unit 232 can be controlled by the processing unit 220 and it may use the memory capacity of the memory unit for storing measurement parameters, for instance.

In an embodiment, the performance monitor communication unit 230 includes a radio transceiver, which may be based on the Bluetooth technology.

In an embodiment, the performance monitor communication unit 230 includes an audio signal transceiver.

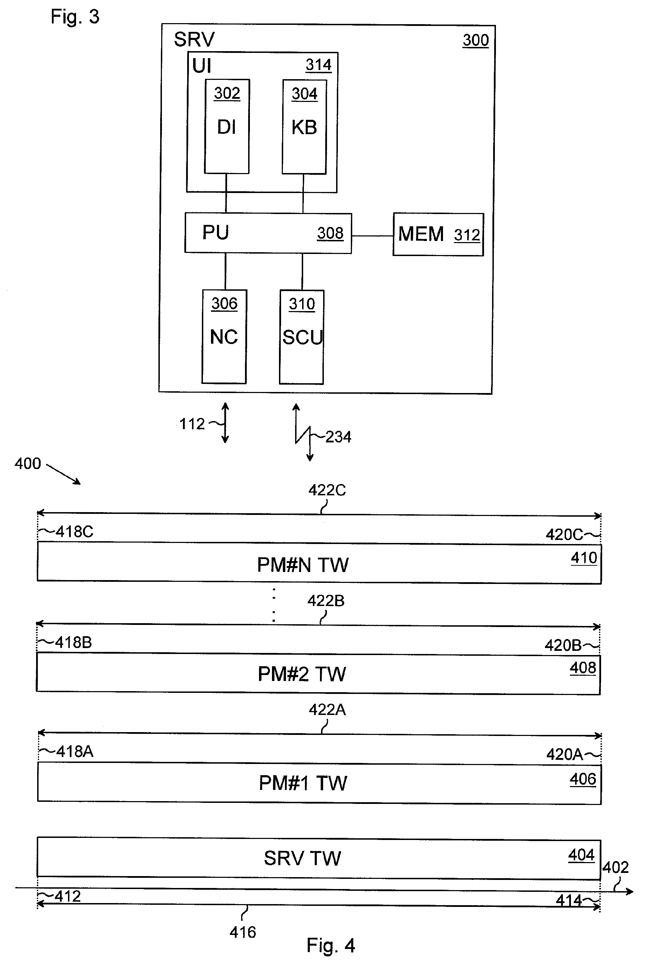

Referring to the example of Figure 3, the server (SRV) 300 comprises a display unit (DI) 302, a keyboard (KB) 304, a network communication unit (NC) 306, a processing unit (PU) 308, a server communication unit (SCU) 310 and a memory unit (MEM) 312.

The display unit 302 and the keyboard 304 are included in the user interface 314 of the server 300, which enables the operator to monitor server outputs and feed data and commands into the server. In some embodiments, the display unit 302 and keyboard 304 are integrated into a touch screen. The user interface 314 may further include a graphic cursor controller, such as a mouse.

The processing unit 308 includes a digital processor for managing the system 100. The processing related to the system 100 management comprises, for example, processing of training information and controlling of protocols related to the transfer of training information.

The memory unit 312 may include software to be executed in the processing unit 308. The memory unit 312 further includes training information.

The network communication unit 306 implements a network connection 112 between the server 300 and the external network 108. If the network connection 112 is a wireless connection, the network communication unit 306 includes a radio modem for implementing the wireless connection. If the network connection 112 is a fixed network connection, the network communication unit 306 typically includes a fixed modem and/or a network interface unit, such as a network interface card.

The server communication unit 310 implements a data transmission connection 234 and communicates training information with the performance monitors 104A to 104C using the data transmission connection 234 and performance monitor-specific identifiers.

The memory unit 312 of the server 300 may include computer software comprising encoded instructions for communicating training information with the performance monitors 104A to 104C of the group of performance monitors using the data transmission connection 106A to 106C and performance monitor-specific identifiers as well as encoded instructions for keeping the server communication unit 310 in a first stand-by mode for a predetermined time window 404, 504 of the server for establishing the data transmission connection 106A to 106C.

In an embodiment, the server communication unit 310 includes an infrared transceiver. The infrared transceiver may support a standardized protocol, such as the IrDA.

In an embodiment, the server communication unit 310 includes a radio transceiver, which may be based on the Bluetooth technology, for instance.

In an embodiment, the server communication unit 310 includes an audio signal transceiver.

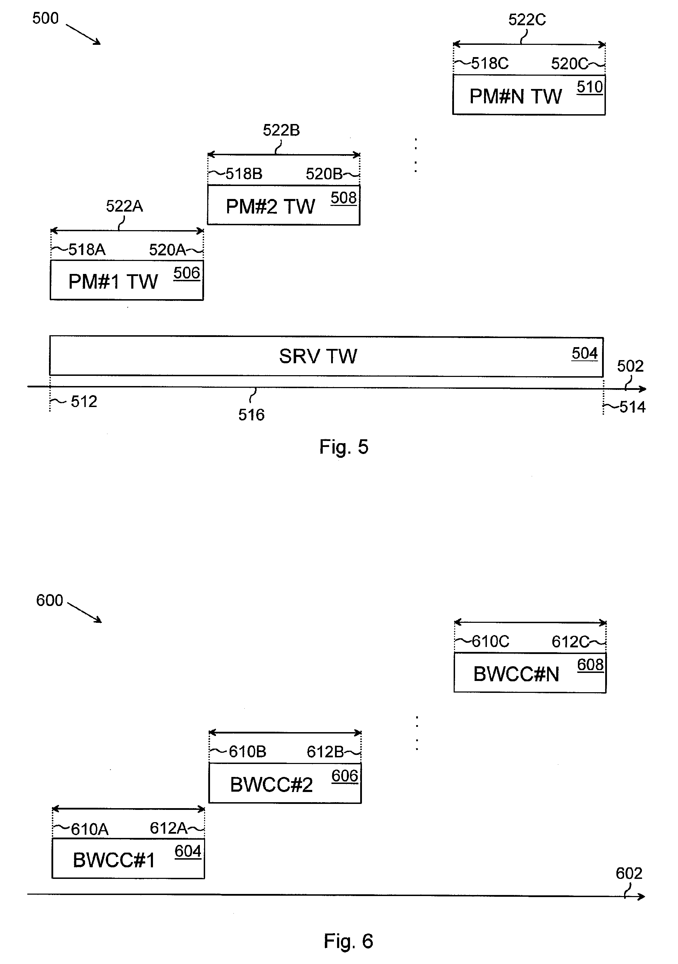

Examples of the server time window (SRV TW) 404, 504 and the performance monitor time window (PM#1 TW, PM#2 TW, PM#N TW) 406, 408, 410, 506, 508, 510 are examined with reference to timing charts 400, 500 in Figures 4 and 5. The horizontal axes 402 and 502 represent time as a time unit, such as a minute. In the examples of Figures 4 and 5, each performance monitor time window 406 to 410, 506 to 510 is associated with one performance monitor 104A to 104C. Thus N performance monitors correspond to N performance monitor time windows 406 to 410, 506 to 510.

The server time window 404, 504 is the time the server communication unit 310 is in the first stand-by mode for establishing the data transmission connection 106A to 106C. In that case, at least the initiation time limit 412 of the server time window 404, 504 is predetermined. In an embodiment, the expiry time limit 414, 514 of the server time window 404, 504 is also predetermined. In that case, the duration 416 of the server time window 404, 504 is predetermined. The server time window 404, 504 can be predetermined so that the operator, for example, programs the time limits defining the server time window 404, 504, such as the initiation time limit 412, 512 and/or the expiry time limit 414, 514 and/or the duration 416, 516, into the memory unit 312 of the server 300. The server processing unit 308 may compare its clock with the time limits 412, 512, 414, 514 in the memory unit 312 and set the server communication in the first stand-by mode or to set it to exit the first stand-by mode when the time limit is reached.

The first stand-by mode is a mode where the server communication unit 310 is ready to start establishment of the data transmission connection 106A to 106C. If the data transmission connection 106A to 106C is established, the server communication unit 310 may exit the stand-by mode.

The performance monitor time window 406 to 410, 506 to 510 is the time the performance monitor 104A to 104C is in the second stand-by mode for establishing the data transmission connection 106A to 106C. The performance monitor time window 406 to 410, 506 to 510 temporally overlaps at least partly with the server time window 404, 504. In that case, a moment exists which is within the predetermined time limits and during which both the performance monitor 104A to 104C and the server 102 are simultaneously in the stand-by mode for establishing the data transmission connection 106A to 106C.

The second stand-by mode is a mode where the performance monitor communication unit 230 is ready to start establishing the data transmission connection 106A to 106C. If the data transmission connection 106A to 106C is established, the performance monitor communication unit 230 may exit the stand-by mode.

The performance monitor time window 406 to 410, 506 to 510 is predetermined. In that case, at least the initiation time limit 418A to 418C, 518A to 518C of the performance monitor time window 406 to 410, 506 to 510 is predetermined. In an embodiment, the expiry time limit 420A to 420 C of the performance monitor time window 406 to 410, 506 to 510 is also predetermined. In that case, the duration 422A to 422 C, 522A to 522C of the performance monitor time window 406 to 410, 506 to 510 is also predetermined.

The time limits of the performance monitor time window 406 to 410, 506 to 510, such as the initiation time limit 418A to 418A, 518A to 518C and the expiry time limit 420A to 420C, 520A to 520C, may be stored in the memory unit 222 of the performance monitor 204. The processing unit 220 of the performance monitor 204 may compare the time of its clock with the time limits in the memory unit 222 and set the performance monitor communication unit 230 to a second stand-by mode or set it to exit the second stand-by mode after the time limit has been reached.

The use of predetermined and overlapping time windows enables timed data transmission between the server 102 and the performance monitors 104A to 104C. This provides power savings in the performance monitors 104A to 104C and in the server 102 since the data transmission connection 106A to 106 does not use resources between the stand-by modes. Furthermore, timed data transmission can be used for distinguishing training information between different user groups because timing as such includes information on the user group. Such a situation is feasible in the case of school sports groups, for example, because the physical education of sports groups takes place regularly in a predeterminable manner.

The initiation time limit 418A to 418C, 518A to 518C of the performance monitor time window 406 to 410, 506 to 510 can be timed, for example, to the finishing time of a performance or to the time following the finishing moment, for example, when it is to be expected that the users can return the performance monitors 104A to 104C they have used to the operator, or they are otherwise located in the proximity of the server 102 so that the data transmission connection 106A to 106C can be established. The duration 416, 516 of the server time window may be, for example, 5 to 15 minutes without being limited thereto. The duration 422A to 422C, 522A to 522C of the time window of the performance monitor may be, for example, 1 to 5 minutes without being limited to this.

In an embodiment, the second stand-by mode is a reception mode and the first stand-by mode is a transmission mode. In that case, the receiver of the performance monitor communication unit 230 is typically active, waiting for an initiative from the server 300 communication unit 310 for establishing the data transmission connection 234. The server communication unit 310 is typically in the transmission mode, in which case the server communication unit 310 transmits the initiation signals required by the data transmission connection 234 to the performance monitor 204. In this case, the time windows may follow a timing similar to the one illustrated in Figure 4, where the time windows 406 to 410 of different performance monitors 104A to 104C overlap with one another and where the initiation time limits 418A to 418C of the time windows 406 to 410 of the performance monitors 104A to 104C are substantially the same as the initiation time limit 412 of the server time window 404. The data transmission connection 106A to 106C can be established, for example, so that the server communication unit 310 alternately establishes a connection to each performance monitor 104A to 104C and/or gives the performance monitors 104A to 104C instructions to establish the data transmission connection 106A to 106C. The instructions may include timing instructions, according to which each performance monitor 104A to 104C may later activate data transmission protocols.

In another embodiment, the second stand-by mode is a transmission mode and the first stand-by mode is a reception mode. In that case, the receiver of the server communication unit 310 is active, waiting for an initiative from the performance monitor 104A to 104C for establishing the data transmission connection 106A to 106C. The performance monitor communication unit 230 is typically in the transmission mode, where the performance monitor 104A to 104C transmits the initiation signals required by the data transmission connection 234 to the server. In this case, the time windows of the performance monitors may be timed as illustrated in Figure 5, where the time windows 506 to 510 of the performance monitors are temporally separated from one another, and thus the signals transmitted from different performance monitors 104A to 104C are not mixed in the server 102.

In an embodiment, the server sets the time limits 418A to 418C, 518A to 518C, 420A to 420C, 520A to 520C for the predetermined time window 406 to 410, 506 to 510 of the performance monitor in the performance monitors 104A to 104C. The operator may set the time limits 412, 414, 512, 514 for the server time window 404, 504 in the server 102, on the basis of which the server processing unit 308 may define the time limits 418A to 418C, 518A to 518C, 420A to 420C, 520A to 520C for the performance monitor. The time limits 418A to 418C, 518A to 518C, 420A to 420C, 520A to 520C can be set according to encoded instructions, which are stored in the server 300 memory unit 312 and executed in the server 300 processing unit, and the time limits can be transmitted to the performance monitors 104A to 104C using the data transmission connection 106A to 106C.

An example of timing data transmission will be examined with reference to a timing chart 600 in Figure 6. The horizontal axis 602 represents time in seconds, for instance.

In an embodiment, the performance monitor communication unit 230 and the server communication unit 310 communicate training information with each other automatically according to predetermined timing after the data transmission connection 106A to 106C has been established. For the embodiment described, the server 300 memory unit 312 may include encoded instructions, which are executed in the processing unit 318. The example of Figure 6 illustrates data transmission connections (BWCC#1, BWCC#3,..., BWCC#N) 604, 606, 608 between the server 102 and the performance monitor 104A to 104C, which correspond to the data transmission connections 106A to 106C of Figure 1.

In automatic communication of training information, the performance monitor communication unit 230 and the server communication unit 310 transfer training information without active measures taken by the user, such as establishment of a data transmission connection and timing of different data transmission connections 106A to 106C. The user may, however, monitor the communication of training information and, if necessary, control it.

Timing of the training information communication is determined by the time limits of each data transmission connection, which are typically an initiation time limit 610A, 610B, 610C and an expiry time limit 612A, 612B, 612C. In the example of Figure 6, the data transmission connections 604 to 608 and communication of training information are performed alternately on each performance monitor 104A to 104C.

In an embodiment, the server 102 acknowledges that the performance monitor 104A to 104C that has established the data transmission connection 106A to 106C with the server has been returned. The establishment of the data transmission connection 106A to 106C requires that the performance monitor 104A to 104C be located within the coverage of the data transmission connection 106A to 106C from the server 102 during the performance monitor time window 406 to 410, 506 to 510. Considering that the data transmission connection 106A to 106C is typically a local area connection, the establishment of the data transmission connection 106A to 106C should be regarded as a sign that the performance monitor 104A to 104C has been returned. The acknowledgement of returning can be shown to the user on the display unit 302 of the server 102.

The server 102 may include an identifier register of the performance monitors 104A to 104C managed by it. After the data transmission connection 106A to 106C has been established, the server 102 may compare the identifier of the performance monitor in the data transmission connection 106A to 106C to identifiers in the identifier register, and thus the server 102 is aware of both the returned and the unreturned performance monitors 104A to 104C. Consequently, the server 102 may also report on unreturned performance monitors 104A to 104C to the operator.

In an embodiment, the performance monitor 104A to 104C informs its user if the quality of the data transmission connection 106A to 106C is below a predetermined level after a predetermined time window has expired. The predetermined time window may be the performance monitor time window 406 to 410, 506 to 510. The fact that the quality is below the predetermined level may mean that no data transmission connection 106A to 106C has been established or that the performance monitor 104A to 104C is outside the coverage of the data transmission connection 106A to 106C. Informing the user that the quality is below the predetermined level reminds the user that the performance monitor should be returned to the operator.

The performance monitor communication unit 230 may, for example, measure the signal transmitted by the server 102 and inform the processing unit 220 of the measurement results. The processing unit 220 may compare the measurement results with limit values in the memory unit 222, for example, and, if necessary, start a process which informs the user if the quality of the data transmission connection 106A to 106C is below a predetermined level.

One aspect of the solution described relates to a computer software product which includes encoded instructions for executing a computer process in a server 102. The encoded instructions may be stored in the memory unit 312 of the server 300 and executed in the processing unit 308 of the server 300.

Another aspect of the solution described relates to a computer software product which includes encoded instructions for executing a computer process in a performance monitor 114. The encoded instructions may be stored in the memory unit 120 of the performance monitor 114 and executed in the processing unit 118 of the performance monitor 114 and/or in the communication unit 122 of the performance monitor 114.

The computer software product may be stored on a computer program distribution medium. The distribution medium may be any known medium for distributing a computer program from the producer/retailer to the end user. The distribution medium may be, for example, a medium readable by a data processing device, a program storage medium or a storage medium, a memory readable by a data processing device or a program distribution package, and a signal decodable by a data processing device, a telecommunication signal or a compressed software package.

Even though the invention was described above with reference to the example according to the accompanying drawings, it is clear that the invention is not limited thereto but it may be modified in various ways within the scope of the appended claims.

## Claims

1. A system for processing training information on a user group of performance monitors, comprising:
at least one user-specific performance monitor (104A to 104C) for registering training information, the at least one performance monitor (104A to 104C) comprising a performance monitor communication unit (102), which is configured to communicate training information using a two-way wireless data transmission connection, and at least one performance monitor-specific identifier being associated with the at least one user-specific performance monitor (104A to 104C); and
at least one server (102) for managing the performance monitor (104A to 104C), the at least one server (102) comprising a server communication unit (310), which is configured to communicate training information with at least one user-specific performance monitor (104A to 104C) using a two-way wireless data transmission connection and performance monitor-specific identifiers, **characterized in that**
the server communication unit (310) is configured to be in a first stand-by mode for a pre-determined time window of the server for establishing the two-way wireless data transmission connection; and
the performance monitor communication unit (230) is configured to be in a second stand-by mode for a pre-determined time window of the performance monitor for establishing the two-way wireless data transmission connection, the predetermined time window of the performance monitor temporally overlapping at least partly with the predetermined time window of the server.

2. A system according to claim 1, **characterized in that** the first stand-by mode and the second stand-by mode form a combination comprising at least one of the following: the second stand-by mode is a reception mode and the first stand-by mode is a transmission mode; the second stand-by mode is a transmission mode and the first stand-by state is a reception mode.

3. A system according to claim 1, **characterized in that** the server (102) is configured to acknowledge that the performance monitor (104A to 104C) that has established the two-way data transmission connection has been returned.

4. A system according to claim 1, **characterized in that** at least one user-specific performance monitor (104A to 104C) is configured to inform its user if the quality of the two-way data transmission connection is below a predetermined level before a predetermined time window has expired.

5. A system according to claim 1, **characterized in that** the performance monitor communication unit (230) and the server communication unit (310) are configured to communicate training information with each other automatically according to predetermined timing after the data transmission connection has been established.

6. A system according to claim 1, **characterized in that** the server (102) is configured to set time limits for a predetermined performance monitor time window in at least one performance monitor (1 04A to 104C).

7. A user-specific performance monitor for registering training information on a user group, the user-specific performance monitor belonging to a group of performance monitors and comprising a performance monitor communication unit (230), which is configured to communicate training information with a server managing the group of performance monitors using a two-way wireless data transmission connection, and a performance monitor-specific identifier being associated with the user-specific performance monitor to distinguish the user-specific performance monitor from the other performance monitors, **characterized in that** the performance monitor communication unit (230) is configured to be in a second stand-by mode for a predetermined time window of the performance monitor for establishing the two-way wireless data transmission connection, the predetermined time window of the performance monitor temporally overlapping at least partly with a predetermined time window of the server, the predetermined time window of the server being the time the server is in a first stand-by mode for establishing the two-way data transmission connection.

8. A user-specific performance monitor according to claim 7, **characterized in that** the second stand-by mode is at least one of the following: a reception mode, whereby the first stand-by mode is a transmission mode; a transmission mode, whereby the first stand-by mode is a reception mode.

9. A user-specific performance monitor, **characterized in that** the user-specific performance monitor is configured to inform its user if the quality of the two-way data transmission connection is below a predetermined level after a predetermined time window has expired.

10. A user-specific performance monitor according to claim 7, **characterized in that** the performance monitor communication unit (230) is configured to communicate training information with the server automatically according to predetermined timing after the data transmission connection has been established.

11. A user-specific performance monitor according to claim 7, **characterized in that** the performance monitor communication unit (230) is configured to set time limits for the predetermined performance monitor time window according to server instructions.

12. A server for managing a group of performance monitors, comprising a server communication unit (310) configured to communicate training information with a user-specific performance monitor of the group of performance monitors using a two-way wireless data transmission connection and a performance monitor-specific identifier, **characterized in that**
the server communication unit (310) is configured to be in a first stand-by mode for a predetermined time window of the server for establishing the two-way data transmission connection, the predetermined time window of the server temporally overlapping at least partly with a predetermined time window of the performance monitor, the predetermined time window of the performance monitor being the time the user-specific performance monitor is in a second stand-by mode for establishing the two-way wireless data transmission connection.

13. A server according to claim 12, **characterized in that** the first stand-by mode and the second stand-by mode form a combination comprising at least one of the following: the second stand-by mode is a reception mode and the first stand-by mode is a transmission mode; the second stand-by mode is a transmission mode and the first stand-by mode is a reception mode.

14. A server according to claim 12, **characterized in that** the server (102) is configured to acknowledge that the performance monitor (1 04A to 104C) that has established the two-way wireless data transmission connection has been returned.

15. A server according to claim 12, **characterized in that** the server (102) is configured to communicate training information with the user-specific performance monitor automatically according to predetermined timing after the data transmission connection has been established.

16. A server according to claim 12, **characterized in that** the server (102) is configured to set time limits for the predetermined performance monitor time window in the performance monitor (1 04A to 104C).

17. A computer software product for managing a group of performance monitors, the computer software product being incorporated into a distribution medium and including encoded instructions for communicating training information between a user-specific performance monitor of the group of performance monitors and the server using a two-way wireless data transmission connection and performance monitor-specific identifiers, **characterized in that** the computer software product further comprises instructions for keeping the performance monitor in a second stand-by mode for a predetermined time window of the performance monitor, the predetermined time window of the performance monitor temporally overlapping at least partly with a predetermined time window of the server, the predetermined time window of the server being the time the server is in a first stand-by mode for establishing the two-way wireless data transmission connection.

18. A computer software product according to claim 17, **characterized in that** the second stand-by mode is at least one of the following: a reception mode, whereby the first stand-by mode is a transmission mode; a transmission mode, whereby the first stand-by mode is a reception mode.

19. A computer software product according to claim 17, **characterized in that** the computer software product further comprises encoded instructions for informing its user if the quality of the two-way data transmission connection is below a predetermined level after a predetermined time window has expired.

20. A computer software product according to claim 17, **characterized in that** the computer software product further comprises encoded instructions for communicating training information with the server automatically according to predetermined timing after the data transmission connection has been established.

21. A computer software product according to claim 17, **characterized in that** the computer software product further comprises encoded instructions for setting time limits for the predetermined performance monitor time window according to server instructions.

22. A computer software product for managing a group of performance monitors, the computer software product being incorporated into a distribution medium and including encoded instructions for communicating training information between a user-specific performance monitor of the group of performance monitors and a server using a two-way wireless data transmission connection and performance monitor-specific identifiers, **characterized in that** the computer software product further comprises instructions for keeping the performance monitor in a first stand-by mode for a predetermined time window of the server, the predetermined time window of the server temporally overlapping at least partly with a predetermined time window of the performance monitor, the predetermined time window of the performance monitor being the time the user-specific performance monitor is in a second stand-by mode for establishing the two-way wireless data transmission connection.

23. A computer software product according to claim 22, **characterized in that** the first stand-by mode and the second stand-by mode form a combination comprising at least one of the following: the second stand-by mode is a reception mode and the first stand-by mode is a transmission mode; the second stand-by mode is a transmission mode and the first stand-by mode is a reception mode.

24. A computer software product according to claim 22, **characterized in that** the computer software product further comprises encoded instructions for acknowledging that the performance monitor that has established the two-way wireless data transmission connection has been returned.

25. A computer software product according to claim 22, **characterized in that** the computer software product further comprises encoded instructions for communicating training information with the user-specific performance monitor automatically according to predetermined timing after the data transmission connection has been established.

26. A computer software product according to claim 22, **characterized in that** the computer software product further comprises encoded instructions for setting time limits for the predetermined performance monitor time window in the performance monitor.
